# EUROPEAN PATENT APPLICATION

(11) **EP 3 494 987 A1**
(43) Date of publication of application: **12.06.2019**
(21) Application number: 17206513.8
(22) Date of filing: 11.12.2017
(51) Int. Cl.: A61K 38/45, A61K 48/00

(54) **COMPOSITIONS FOR THE TREATMENT OR PREVENTION OF NEURODEGENERATIVE DISORDERS, IN PARTICULAR PARKINSON`S DISEASE**

(71) Applicant: Fraunhofer-Gesellschaft zur Förderung der angewandten Forschung e.V., 80686 München (DE)
(72) Inventor: WAGNER, Sylvia, 66280 Sulzbach (DE); STAB, Julia, 66280 Sulzbach (DE); VON BRIESEN, Hagen, 66280 Sulzbach (DE)
(74) Representative: v. Bezold & Partner Patentanwälte - PartG mbB

(57) **Abstract**

The present invention relates to a pharmaceutical composition for the treatment or prevention of neurodegenerative disorders, in particular neuro-degenerative disorders which involve elevated levels of reactive oxygen species (ROS) in neuronal cells, comprising a delivery system which comprises a nucleic acid sequence coding for a telomerase, in particular human telomerase (hTERT), or the corresponding telomerase protein, as well as the delivery system for use in the treatment of such diseases.

The neuro-degenerative disorder may be, e.g., selected from the group comprising Alzheimer's disease, Huntington's disease, Creutzfeldt-Jakob's disease, amyotrophic lateral sclerosis (ALS) and Parkinson's disease.

The delivery system of said pharmaceutical composition may comprise a functional telomerase protein, in particular a hTERT protein, as well as a carrier, and/or a nucleic acid sequence coding for a telomerase, in particular human telomerase (hTERT), which may be DNA, mRNA or a derivative thereof, as well as a carrier or vector, in particular selected from the group comprising viral vectors and non-viral vectors.

Non-viral vectors may be, e.g., selected from the group comprising nanoparticles, liposomes, polymers, (poly)peptides, DNA nanostructures, plasmids, and minicircle DNA. Viral vectors may be in particular selected from the group comprising retroviral vectors such as adenoviruses, lentiviruses, other viral vectors such as HSV, Sendai virus, poxviruses, papilloma viruses and parvoviruses, in particular variants of adeno-associated virus (AAV).

In one specific embodiment, the delivery system comprises an expression vector construct including an expression cassette comprising a functional telomerase gene as well as any regulatory sequences required for its expression.

## Description

### Background of the invention

Parkinson's disease (PD) is one of the most common neurodegenerative disorder and was first described by James Parkinson in 1817 [Goetz CG., The history of Parkinson's disease: early clinical descriptions and neurological therapies. Cold Spring Harb Perspect Med. 2011 Sep;1(1):a008862.].

PD is characterized by the selective, extensive loss of dopaminergic neurons in the brain region of the *Substantia nigra pars compacta.* At the time of the diagnosis a large proportion of the dopaminergic neurons are already lost [Vernier P, Moret F, Callier S, Snapyan M, Wersinger C, Sidhu A., The degeneration of dopamine neurons in Parkinson's disease: insights from embryology and evolution of the mesostriatocortical system. Ann N Y Acad Sci. 2004 Dec;1035:231-49.].

The degeneration of this special population of dopamine synthesizing neurons results in a lack of dopaminergic regulatory input for neurons in the brain region of the *striatum*, prefrontal cortex and basal ganglia and finally interferes with normal motor function, including the impaired elicitation of automatic behaviors and responses to novelty. During the progression of the disease, three cardinal symptoms, namely akinesia, rigor and tremor, appear [Béné R, Antic S, Budisić M, Lisak M, Trkanjec Z, Demarin V, Podobnik-Sarkanji S., Parkinson's disease. Acta Clin Croat. 2009 Sep;48(3):377-80.].

The mechanisms that cause Parkinson's disease are currently a main focus of discussion. Protein aggregation (as also described as a factor for the formation of Alzheimer's disease) hypothesis include the agglutination of a protein called alpha-synuclein. Malfunctions of the proteasome or the cytoskeleton are also discussed.

In particular, the development of reactive oxygen species (ROS) is assumed to play a major role in neurodegenerative disorders such as Parkinson's disease, Alzheimer's disease, Huntington's disease, Creutzfeldt-Jakob's disease, amyotrophic lateral sclerosis (ALS) and others [Tellone, E. et al., How does resveratrol influence the genesis of some neurodegenerative diseases?. Neural Regen Res 11(1):86-87; Hald, A. & Lotharius,T Oxidative stress and inflammation in Parkinson's disease: Is there a causal link? Experimental Neurology 193 (2005) 279-290.] For Parkinson's disease it is known that the metabolism of dopamine itself results in major oxidative stress inducers in cells. In Parkinson's disease patients either the level of the involved reactive oxygen species (ROS) could be elevated or the normal counter processes might be decreased.

This assumption is supported by the fact that various toxins that cause PD-like characteristics also act via elevated ROS levels in animal models and in humans. For example MPTP, a byproduct in the unsuccessful synthesis of MPPP (a neuroactive drug), was reported to cause severe symptoms of PD that could be treated successfully with the standard PD medication Levodopa. MPTP is transported to the brain across the blood-brain barrier and converted to MPP+ by glia cells. The MPP+ then is actively and selectively taken up by dopaminergic neurons by dopamine transporters and elevated neurotoxic ROS levels occur, leading to lipid peroxidation, protein peroxidation, DNA damage and eventually to cellular death.

Today, causal interventions for Parkinson's disease are still lacking, but desperately needed. The standard medication focusses on compensating the depleted levels of dopamine in order to improve dopamine stimuli. Pharmacologically, the lack of dopamine in Parkinson's disease is treated by administration of the prodrug levodopa (L-Dopa). Levodopa is able to bypass the blood-brain barrier and will be metabolized to dopamine in the brain. In order to prevent the degradation of levodopa in the body, decarboxylase inhibitors such as carbidopa or catechol-O-methyl transferase inhibitors are co-applied. Levodopa-carbidopa combination therapy is still the most efficient way of treatment, but as the degeneration of dopaminergic neurons cannot be stopped by this medication, the symptomatic improves for a certain amount of time (usually several years), but cannot stop the ongoing neurodegeneration. Finally, when the population of dopaminergic neurons shrinks underneath a certain threshold, Levodopa does not improve symptoms anymore and patients suffer from dyskinesia after 5 to 10 years of levodopa-carbidopa treatment [Fernandez HH, 2015 Update on Parkinson disease., Cleve Clin J Med. 2015 Sep;82(9):563-8.].

Therefore, a main object of the present invention was to provide novel and improved means for the treatment and/or prevention of neurodegenerative disorders, in particular Parkinson's disease.

This objective has been achieved by providing the composition according to present claim 1. More specific and/or preferred embodiments of the invention are the subject of further claims.

### Description of the invention

In view of the above discussed role of reactive oxygen species (ROS) in Parkinson's disease, the present inventors focused on a new approach for the treatment and/or prevention of Parkinson's disease, namely to counteract ROS induced cellular damages in order to protect from the degeneration of dopaminergic neurons and found that the presence of a telomerase, in particular human telomerase (hTERT), in such neuronal cells actually resulted in a reduction of oxidative stress in model cells for Parkinson's disease (PD).

Consequently, in one aspect the present invention provides a pharmaceutical composition for the treatment or prevention of neurodegenerative disorders such as Parkinson's disease, Alzheimer's disease, Huntington's disease, Creutzfeldt-Jakob's disease, amyotrophic lateral sclerosis (ALS) and others, in particular neuro-degenerative disorders which involve elevated levels of reactive oxygen species (ROS) in neuronal cells, specifically Parkinson's disease, which composition comprises a delivery system comprising a nucleic acid sequence coding for a telomerase, in particular human telomerase (hTERT), or the corresponding telomerase protein.

The term "telomerase" principally refers to any telomerase enzyme, in particular any vertebrate or, more specifically, any mammalian telomerase. Preferably, the telomerase is human telomerase.

A telomerase, such as human telomerase (hTERT), is a specialized reverse transcriptase that synthesizes telomeric DNA repeats to the ends of chromosomes. Telomeres are shortened during cell division based on a semi-conservative replication that leads to a loss of genetic information and aging of cells. Telomerase prevents shortening of telomeres by a still poorly understood procedure. For catalytic activity, the ribonucleoprotein complex requires two key subunits: telomerase reverse transcriptase subunit (in particular hTERT) and an intrinsic RNA (hTR) that serves as template for adding multiple G-rich repeats to the 3'end of telomeres. Due to the repeated usage of telomerase specific RNA as template, the nucleic acid is not degraded after transcription. This enzymatic mode of action significantly differs from viral reverse transcriptases.

The protein human telomerase (hTERT) is generally known for its function in the elongation of telomers. Independent from this classical hTERT function, the protein was also reported to have a plurality of extratelomeric functions in a variety of cell types including the prevention of neuronal apoptosis induced by cell-damaging agents such as H₂O₂, TNF, TRAIL, etoposide and ROS [Chung, H. K., et al. Current Molecular Medicine 5 (2005) 233-241)]. Said reference, however, did neither disclose nor suggest any application of hTERT in the treatment or prevention of Parkinson's disease or other neurodegenerative disorders.

The delivery system of the claimed pharmaceutical composition is not especially limited and may be principally any delivery system which is able to provide for the presence of a functional telomerase in the target cells, in particular neuronal target cells.

In one embodiment, the delivery system comprises a functional telomerase protein, in particular a hTERT protein, and optionally also any telomerase RNA subunit, such as the intrinsic RNA of hTERT (TR), as well as a suitable carrier, in particular selected from the group comprising nanoparticles, including inorganic nanoparticles, e.g. silver, gold, iron oxide or silica nanoparticles, protein-based nanoparticles, e.g. human serum albumin nanoparticles, protamine nanoparticles, polysaccharide-based nanoparticles, e.g. chitosan nanoparticles, alginate nanoparticles, polymer nanoparticles, lipid nanoparticles, liposomes, polymers, in particular cationic polymers, including polysomes and polymeric vesicles, peptide-modified polymers, polypeptides, including dendronized polypeptides, cell-penetrating peptides, such as CPP.

In another, more typical embodiment, the delivery system comprises a nucleic acid sequence coding for a telomerase, in particular human telomerase (hTERT), which may comprise DNA, mRNA or a derivative thereof, as well as a suitable carrier or vector.

A "nucleic acid sequence coding for a telomerase" includes at least a nucleic acid template coding for the telomerase reverse transcriptase subunit and optionally also a DNA template coding for the intrinsic telomerase RNA (TR) and/or the TR RNA as such.

In one typical embodiment, both a nucleic acid template coding for the telomerase reverse transcriptase subunit as well as a DNA template coding for the intrinsic telomerase RNA (TR) or the TR RNA as such are included.

The terms "telomerase" and in particular "functional telomerase" or "functional telomerase protein" as used herein include or refer to a telomerase protein which is at least capable to perform its - in this context- essential function as a neuronal cell protecting agent, in particular in the treatment of degenerative neuronal disorders such as PD. More specifically, the term "functional telomerase protein" includes a telomerase protein having its native peptide sequence (which at least in the case of hTERT is known and available from several databases) as well as any modifications or derivatives thereof which do not essentially impair its telomerase and/or extratelomeric functions, in particular the desired cell-protective function. For example it is well-known to effect conservative substitutions of one or more amino acids in a given sequence of a protein or peptide without essentially impairing its properties and functions. Also, it may be possible to delete or modify (e.g. methylate) one or more amino acids without essentially impairing its properties and functions.

Consequently, such derivatives or variants of a telomerase protein are still encompassed by the terms "telomerase" or "functional telomerase" as used herein and the term "nucleic acid sequence coding for a telomerase" also encompasses nucleic acid sequences coding for such functional variants or derivatives of a telomerase protein. Similarly, the DNA template coding for the intrinsic telomerase RNA (TR) and/or the TR RNA as such may also code for or include functional variants or derivatives of the TR RNA.

The carrier or vector in this embodiment is not especially limited and may be selected from a wide variety of known vectors or carriers for gene delivery, in particular targeted gene delivery, into cells.

Generally, the vector or carrier may be selected from the group comprising viral vectors and non-viral vectors.

Non-viral vectors may be, e.g., selected from the group comprising nanoparticles, including inorganic nanoparticles, e.g. silver, gold, iron oxide or silica nanoparticles, protein-based nanoparticles, e.g. human serum albumin nanoparticles, protamine nanoparticles, polysaccharide-based nanoparticles, e.g. chitosan nanoparticles, alginate nanoparticles, polymer nanoparticles, lipid nanoparticles, liposomes, polymers, in particular cationic polymers, including polysomes and polymeric vesicles, peptide-modified polymers, polypeptides, including dendronized polypeptides, cell-penetrating peptides, such as CPP, DNA nanostructures, plasmids, minicircle DNA, and other non-viral vectors known in the art.

Various transfection lipids for generating suitable lipid nanoparticles or liposomes are commercially available, such as Lipofectamine 2000 or 3000, RNAiMax, but are not limited to these examples.

Positively charged cationic polymers can, e.g., be used to form nanocomplexes with DNA or mRNA through electrostatic interactions. Such complexes provide protection of the enclosed nucleic acid from degradation by nucleases and interaction with undesirable binding partners and, thus, increase the efficiency of delivery. Further, such complexes could be additionally provided with a lipid shell, resulting in structures which are sometimes called lipopolyplexes.

Suitable polymers, in particular cationic polymers, may be, e.g., selected from the group comprising polyamines and polyimines, such polyethylene imine (PEI), poly(R-amino esters) (PBAEs), polymethacrylates, such as poly-N,N-dimethylaminoethylmethacrylate, (PDMAEMA) and copolymers and grafted derivatives (e.g. grafted with PEG side chains) thereof, poly-lactic acid (PLA), poly(lactic-co-glycolic acid (PLA), but are not limited to these exemplary compounds and classes of compounds.

In some specific embodiments, peptide-modified polymers may also comprise cell recognizing motifs such as RGD, etc.

A plurality of non-viral vectors and approaches for delivering nucleic-based therapeutics which are principally suitable for the delivery system used in the present invention are known in the art and, e.g., disclosed in Biomater. Sci, 2017, 5, 2188-2211, Biomater. Sci, 2017, 5, 2381-2392, *and* Adv. Healthcare Mater. 2017, 1700886.

In another principal embodiment, the delivery system comprises a viral vector, in particular selected from the group comprising retroviral vectors such as adenoviruses, lentiviruses, other viral vectors such as HSV, Sendai virus, poxviruses (MVA vaccinia), papilloma viruses and parvoviruses, in particular variants of adeno-associated virus (AAV).

In more preferred embodiments, the viral vector may be, e.g., selected from AAV-based vectors, adenoviruses and lentiviruses. Various viral vectors from this group have been successfully employed as gene delivery vectors and some vectors are even commercially available and may be modified as desired.

Typically, the delivery system for delivering a nucleic acid coding for a telomerase will not only comprise the coding sequences a such but rather an expression vector construct including an expression cassette comprising a functional telomerase gene as well as any regulatory sequences required for its expression.

In one specific embodiment, the regulatory sequences may comprise an inducible promoter, e.g. a doxycycline-inducible promoter.

Further, said vector construct may also comprise a nucleic sequence coding for a reporter gene as well as any regulatory sequences required for its expression.

The reporter gene is not especially limited and may be any gene whose expression results in a detectable gene product. The gene product may be for example detectable by emission of electromagnetic radiation such as light emission (e.g. fluorescence, luminescence). In this case, the detection may be effected, e.g., visually or using spectroscopic methods.

In some advantageous embodiments, the reporter gene may be Green Fluorescent Protein (GFP), Red Fluorescent Protein (RFP), Cyan Fluorescent Protein (CFP), Yellow Fluorescent Protein (YEP or YPF) or a derivative thereof.

A closely related aspect of the present invention relates to a delivery system which comprises a nucleic acid sequence coding for a telomerase, in particular human telomerase (hTERT), or the corresponding telomerase protein, for use in the treatment or prevention of neurodegenerative disorders, in particular neuro-degenerative disorders which involve elevated levels of reactive oxygen species (ROS) in neuronal cells.

Typically, said delivery system comprises the components as described with respect to the pharmaceutical composition above and defined in any one of claims 4-11.

In particular, said delivery system is for use in the treatment or prevention of a neuro-degenerative disorder selected from the group comprising Alzheimer's disease, Huntington's disease, Creutzfeldt-Jakob's disease, amyotrophic lateral sclerosis (ALS) and Parkinson's disease.

A further aspect of the invention relates to a method for reducing the amount of reactive oxygen species (ROS) in target cells, e.g. neuronal cells, in particular dopaminergic neuron cells, which comprises introducing a delivery system as described above into target cells, and optionally measuring the level of ROS of transduced target cells and non-transduced reference cells, e.g. with an DCFDA assay.

In one embodiment, said method is an *in vitro* method.

### Brief description of the Figures

**Fig. 1** shows the Western Blot analysis of LUHMES and 293T control cells after transduction with either hTERT-GFP-encoding (LUHMES GFP-hTERT) or GFP-encoding retroviral vector (LUHMES GFP). LUHMES control cells were not transduced.
**Fig. 2** shows the Quantitative PCR (qPCR) data for mRNA expression of hTERT. 2-^{ΔΔCt}-Values of the different cell lines for hTERT expression normalized by GADPH expression in 293T control cells.
**Fig. 3** shows the relative fluorescence intensity indicative for oxidative stress level in either GFP- or GFP-hTERT-transduced LUHMES cells by DCFDA assay (abcam Inc.). As oxidative stress inducers either MPP+ or tert-Butyl hydroperoxide (TBHP) were applied.

The following examples illustrate the present invention in more detail, however, without limiting the same to the specific parameters and conditions thereof.

### EXAMPLE 1

### Telomerase expression in dopaminergic neuron model cells (LUHMES)

### LUHMES cells as cellular model for Parkinson's disease

In LUHMES cells, that can be used as a model for dopaminergic neurons [Scholz D., Pöltl D., Genewsky A., Weng M., Waldmann T., Schildknecht S., Leist M., J Neurochem. 2011 Dec;119(5):957-71], oxidative stress can be induced by toxins such as MPTP to serve as a cellular model of Parkinson's disease. Initial tests verified the expression of various dopaminergic neuron markers. Additionally, it was verified that the substance 1-methyl-4-phenylpyridinium (MPP+) can be used as a PD-inducing toxin in LUHMES cells.

### Expression of the protein human telomerase (hTERT) in different model cells

To investigate if the expression of hTERT protein indeed protects from the damages caused by an increase in reactive oxygen species (ROS) levels, the model cells LUHMES (representing a dopaminergic neuron model) was retrovirally transduced with a hTERT-encoding vector that also carries information for green fluorescent protein (GFP) expression (labelled LUHMES GFP-hTERT in the Western blot). Controls comprised an empty retroviral vector (LUHMES GFP) and cells that were not previously transduced (LUHMES control).

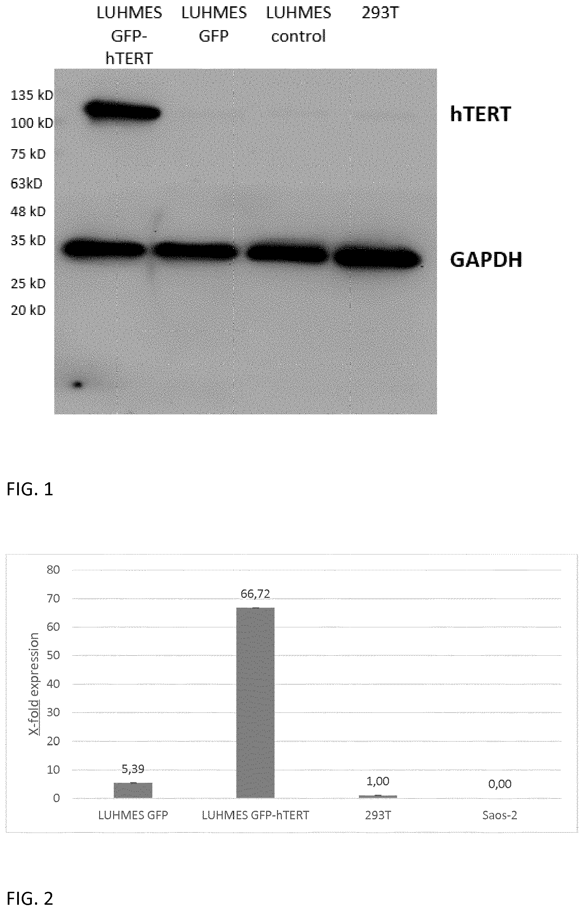

As shown by the Western Blot data of Fig. 1, the expression of a protein between 100 and 135 kDa was detectable in the LUHMES GFP-hTERT, but not in LUHMES GFP or LUHMES control cells. The protein human telomerase (hTERT) is about 128 kDa. Glycerinaldehyd-3-phosphat-dehydrogenase (GAPDH) served as an internal control.

### Verification of hTERT mRNA expression via qPCR experiments

The expression level of hTERT after retroviral transduction was also assessed by quantitative PCR(qPCR).

Fig. 2 shows the Quantitative PCR (qPCR) data for mRNA expression of hTERT. 2-^{ΔΔCt}-Values of the different cell lines for hTERT expression normalized by GADPH expression in 293T control cells.

As expected, the expression of hTERT drastically increased after transducing LUHMES cells with the hTERT-encoding vector (LUHMES GFP-hTERT). 293T cells and Saos-2 served as further control.

### Purification of retrovirally transduced LUHMES cells for further experiments

Both control and hTERT-encoding retroviral vectors also encode for the green fluorescent protein (GFP) to visually allow to determine transduction success. With fluorescence assisted cellular sorting (FACS) the population of transduced LUMES cells was sorted to enhance to proportion of successfully transduced cells for further experiments. For the control vector-transduced LUHMES, the proportion of fluorescent cells increased from 40 % to 92 %. For hTERT-transduced LUHMES cells, the proportion of fluorescent cells increased from 15 % to 83%.

### EXAMPLE 2

### Induction and assessment of oxidative stress responses in hTERT-transduced LUHMES model cells versus control cells

For measuring of ROS in the hTERT-tranduced LUHMES model cells, the cell-permanent 2',7'-dichlorodihydrofluorescin diacetate (DCFDA) assay was used. GFP-and GFP-hTERT transduced LUHMES cells were treated with 50 µM tert-Butyl hydroperoxide (TBHP) to induce oxidative stress.

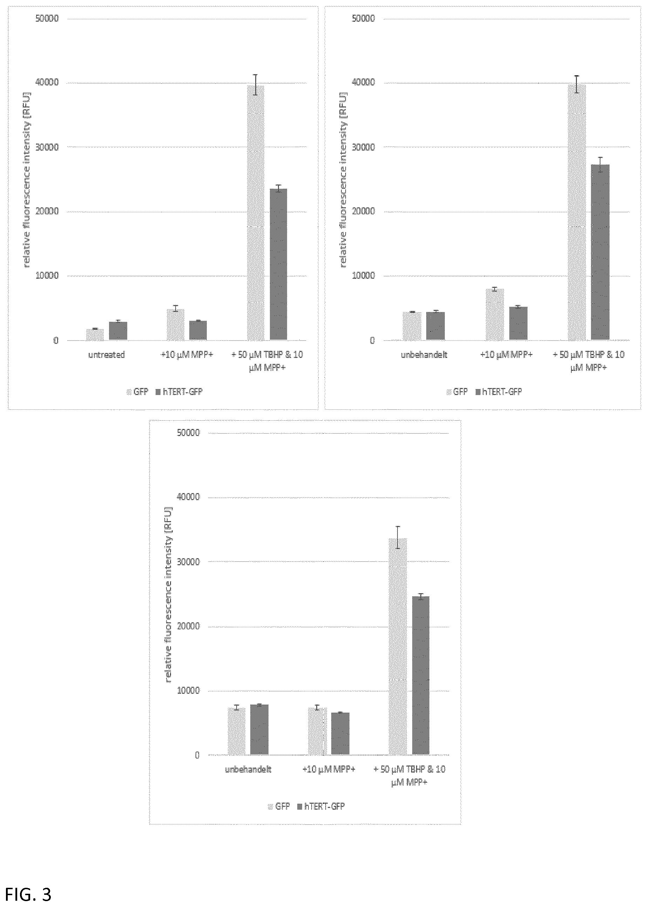

As shown in Fig. 3, the level of ROS was comparable in both GFP- and GFP-hTERT-transduced cells. 10 µM MPP+ application only had very little effect on GFP- and GFP-hTERT-transduced cells alone, but when additionally 50 µM TBHP were added the reactive oxygen stress level differed from GFP- and GFP-hTERT-transduced cells. Control cells transduced only with GFP-protein showed higher ROS levels compared to GFP-hTERT-transduced cells. These data indicate that the expression of hTERT protein in dopaminergic neuron model cells LUHMES can protect from neurotoxin MPP+ induced oxidative stress.

Summarizing, it could be demonstrated that the retroviral transduction of dopaminergic neuron model cells LUHMES with a hTERT-encoding vector and control vectors was successful. hTERT-transduced LUHMES cells showed an increase in hTERT protein expression.

Further, hTERT-transduced LUHMES cells show less oxidative stress responses after exposure to the neurotoxic, PD-inducing MPP+ compared to cells transduced with the control vector without hTERT information hinting at a protective effect of hTERT expression in Parkinson's disease.

## Claims

1. A pharmaceutical composition for the treatment or prevention of neurodegenerative disorders, in particular neuro-degenerative disorders which involve elevated levels of reactive oxygen species (ROS) in neuronal cells, comprising a delivery system which comprises a nucleic acid sequence coding for a telomerase, in particular human telomerase (hTERT), or the corresponding telomerase protein

2. The pharmaceutical composition according to claim 1, wherein the neuro-degenerative disorder is selected from the group comprising Alzheimer's disease, Huntington's disease, Creutzfeldt-Jakob's disease, amyotrophic lateral sclerosis (ALS) and Parkinson's disease.

3. The pharmaceutical composition according to claim 1 or 2, wherein the treatment or prevention of the neurodegenerative disorder, in particular Parkinson's disease, involves the reduction of reactive oxygen species in transduced neuronal target cells.

4. The pharmaceutical composition according to any one of claims 1-3, wherein the delivery system comprises a functional telomerase protein, in particular a hTERT protein, as well as a carrier, in particular selected from the group comprising nanoparticles, including inorganic nanoparticles, e.g. silver, gold, iron oxide or silica nanoparticles, protein-based nanoparticles, e.g. human serum albumin nanoparticles, protamine nanoparticles, polysaccharide-based nanoparticles, e.g. chitosan nanoparticles, alginate nanoparticles, polymer nanoparticles, lipid nanoparticles, liposomes, polymers, in particular cationic polymers, including polysomes and polymeric vesicles, peptide-modified polymers, polypeptides, including dendronized polypeptides, cell-penetrating peptides, such as CPP.

5. The pharmaceutical composition according to any one of claims 1-3, wherein the delivery system comprises a nucleic acid sequence coding for a telomerase, in particular human telomerase (hTERT), which may be DNA, mRNA or a derivative thereof, as well as a carrier or vector, in particular selected from the group comprising viral vectors and non-viral vectors.

6. The pharmaceutical composition according to any one of claims 1-3, wherein the delivery system comprises a non-viral vector, in particular selected from the group comprising nanoparticles, including inorganic nanoparticles, e.g. silver, gold, iron oxide or silica nanoparticles, protein-based nanoparticles, e.g. human serum albumin nanoparticles, protamine nanoparticles, polysaccharide-based nanoparticles, e.g. chitosan nanoparticles, alginate nanoparticles, polymer nanoparticles, lipid nanoparticles, liposomes, polymers, in particular cationic polymers, including polysomes and polymeric vesicles, peptide-modified polymers, polypeptides, including dendronized polypeptides, cell-penetrating peptides, such as CPP, DNA nanostructures, plasmids, minicircle DNA.

7. The pharmaceutical composition according to claim 5, wherein the delivery system comprises a viral vector, in particular selected from the group comprising retroviral vectors such as adenoviruses, lentiviruses, other viral vectors such as HSV, Sendai virus, poxviruses (MVA vaccinia), papilloma viruses and parvoviruses, in particular variants of adeno-associated virus (AAV).

8. The pharmaceutical composition according to claim 5 or 7, wherein the delivery system comprises an expression vector construct including an expression cassette comprising a functional telomerase gene as well as any regulatory sequences required for its expression.

9. The pharmaceutical composition according to claim 8, wherein the vector construct further comprises a nucleic sequence coding for a reporter gene as well as any regulatory sequences required for its expression.

10. The pharmaceutical composition according to claim 8 or 9, wherein the regulatory sequences required for expression of the telomerase gene comprise an inducible promoter, e.g. a doxycycline-inducible promoter.

11. The pharmaceutical composition according to claim 9 or 10, wherein the reporter gene is selected from the group comprising Green Fluorescent Protein (GFP), Red Fluorescent Protein (RFP), Cyan Fluorescent Protein (CFP), Yellow Fluorescent Protein (YEP or YPF) and derivatives thereof.

12. A delivery system which comprises a nucleic acid sequence coding for a telomerase, in particular human telomerase (hTERT), or the corresponding telomerase protein, for use in the treatment or prevention of neurodegenerative disorders, in particular neuro-degenerative disorders which involve elevated levels of reactive oxygen species (ROS) in neuronal cells.

13. The delivery system according to claim 12 which comprises the components as defined in any one of claims 4-11.

14. The delivery system according to claim 12 or 13 for use in the treatment or prevention of a neuro-degenerative disorder selected from the group comprising Alzheimer's disease, Huntington's disease, Creutzfeldt-Jakob's disease, amyotrophic lateral sclerosis (ALS) and Parkinson's disease.

15. A method, in particular an *in vitro* method, for reducing the amount of reactive oxygen species (ROS) in target cells which comprises introducing a delivery system according to one of claims 12-14 into target cells, in particular dopaminergic neuron cells, and optionally measuring the level of ROS of transduced target cells and non-transduced reference cells, e.g. with an DCFDA assay.
